# EUROPÄISCHE PATENTSCHRIFT

(11) **EP 0 682 955 B1**
(45) Veröffentlichungstag und Bekanntmachung des Hinweises auf die Patenterteilung: **05.09.2001**
(21) Anmeldenummer: 94107770.3
(22) Anmeldetag: 19.05.1994
(51) Int. Cl.: A61M 15/00

(54) **Vorrichtung zur Trocknung und Pufferung von Aerosolen**
Device for drying and buffering aerosols
Dispositif pour sécher et amortir l'écoulement d'aérosols

(43) Veröffentlichungstag der Anmeldung: 22.11.1995
(73) Patentinhaber: PARI GmbH Spezialisten für effektive Inhalation, 82319 Starnberg (DE)
(72) Erfinder: Blaha-Schnabel, Ales Dr-Ing, D-90 408 Nürnberg (DE); Knoch, Martin Dr-Ing, D-82 335 Berg (DE)
(74) Vertreter: Zangs, Rainer E., Dipl.-Ing.

(56) Entgegenhaltungen:
- EP-A- 0 504 459
- FR-A- 2 352 556
- US-A- 3 980 074
- US-A- 4 155 359
- US-A- 5 241 954

## Beschreibung

Die Erfindung betrifft eine Vorrichtung zur Trockung und/oder Pufferung von Aerosolen für die Inhalationstherapie.

Die Inhalationstherapie wird nicht nur zur Behandlung von Atemwegserkrankungen, sondern in zunehmendem Maße auch zur Verabreichung anderer medizinischer Wirkstoffe für die systemische Applikation eingesetzt. Bei einer solchen Therapie wird der Wirkstoff als feiner Nebel mit Luft vermischt, um so eingeatmet werden zu können. Wichtig hierbei ist, daß die den Wirkstoff enthaltenden Partikel sehr klein sind (unter 5 µm), um zu gewährleisten, daß sie vom Atemstrom in die Lunge getragen und nicht bereits im Mund- und Rachenraum abgeschieden werden. Erst in der Lunge ist eine effektive und rasche Aufnahme des Wirkstoffs möglich. Die Abscheidung größerer Partikel in Mund oder Rachen kann bei bestimmten Medikamenten auch zu unerwünschten lokalen Nebenwirkungen führen.

Von besonderer Bedeutung sind "nasse Aerosole", worunter solche Aerosole verstanden werden, die durch Verneblung einer Flüssigkeit erzeugt werden, in der der Wirkstoff ggf. zusammen mit einer Trägersubstanz, beispielsweise einem Salz, gelöst oder dispergiert ist. Bei der Verneblung der Flüssigkeit werden feine Tröpfchen gebildet, die im Ausgangsaerosol, also unmittelbar nach der Erzeugung des Aerosols mit Hilfe einer Düse o.ä. noch eine Größenverteilung aufweisen, bei der nur ein bestimmter Anteil der Tröpfchen lungengängig ist. Um den Anteil lungengängiger Tröpfchen zu erhöhen, kann das Aerosol abgetrocknet werden, wodurch die Größe der einzelnen Partikel reduziert wird. Diese Behandlung des Ausgangsaerosols trägt entscheidend zu den letzlich erzielten Eigenschaften des Aerosols bei, das vom Patienten eingeatmet wird. Bei Aerosolen auf Pulverbasis, d.h. bei Aerosolen, die durch Zerstäubung einer pulverförmigen Ausgangssubstanz erzeugt werden, ist die Partikelgröße durch die Ausgangssubstanz festgelegt. Bei beiden Aerosoltypen ist aber die Pufferung einer ausreichenden Aerosolmenge erforderlich.

Um die Ausbildung eines Aeroslos für die Inhaltionstherapie auch der tieferen Atemwege zu unterstützen, ist aus DE 89 08 273 U1 ein Inhalationsgefäß aus Kunstoff bekannt, an dessen amphorenartigen Grundkörper gegenüberliegend an den beiden Stirnseiten ein Einlaß- und ein Auslaßstutzen vorgesehen sind. Durch die amphorenartige Form wird dem Ausgangsaerosol Gelegenheit zur Ausbreitung und Beruhigung gegeben, womit auch eine Abtrocknung einhergeht. Das bekannte Inhalationsgefäß erfüllt die Anforderungen hinsichtlich der Pufferung, d.h. der Bereitstellung eines Vorrats von atembarem Aerosol. Durch Abscheidung und unvollständige Ausräumung des Inhalationsgefäßes gehen aber nicht unerhebliche Teile des Aerosols und damit des Medikaments verloren.

Eine andere Vorrichtung zur Behandlung von Aerosolen ist aus EP 0 504 459 A1 bekannt; die Vorrichtung weist einen zylindrischen Verneblungsraum mit einem Zuführ- und einem Entnahmestutzen auf, die beide jeweils in der Nähe zu einer der Stirnseiten an der Mantelfläche des zylindrischen Verneblungsraumes tangential angeordnet sind. Durch die Anordnung der beiden Stutzen wird ein um die Längsachse des Verneblungsraumes verlaufender Spiralwirbel erzeugt. Dessen Ausbildung kann auch durch ein Kernteil unterstützt werden, das in dem Verneblungsraum koaxial mit dem zylindrischen Verneblungsraum angeordnet ist. Alternativ kann der Zuführstutzen durch eine Wendelfläche im Bereich der einen Stirnseite des Verneblungsraumes ersetzt werden, die bei diffuser Zuführung des Ausgangsaerosols die Ausbildung des Spiralwirbels einleitet. Bei der bekannten Vorrichtung erfolgt eine ausreichende Pufferung von atembarem Aerosol bei gleichzeitiger Homogenisierung und Abtrocknung. Das Volumen eignet sich zur Entleerung durch einen Atemzug. Jedoch neigen die Aerosolpartikel zur Abscheidung an der Innenwand des Verneblungsraumes, insbesondere durch Trägheitskräfte des Naßaerosols unmittelbar nach dem Düsenaustritt oder wenn durch starke Atemzüge die Rotationsgeschwindigkeit erhöht ist und die Zentrifugalkräfte zu groß werden. Neben der aus diesem Grund auftretenden Ablagerung des Wirkstoffes sind im Vernebler Bereiche vorhanden, aus denen das Aerosol nur unvollständig ausgeräumt wird. Letzlich wird durch beide Effekte eine gezielte Beeinflussung der Partikelgröße unmöglich gemacht, zumindest aber erheblich erschwert.

Eine Vorrichtung ähnlichen Aufbaus ist in WO 90/15635 beschrieben; jedoch dient diese Vorrichtung zur Zerstäubung von Partikeln eines bereits in Pulverform vorliegenden Inhalationsmedikaments. Das Medikament wird als trockenes Aerosol, nämlich als feiner Nebel der Pulverpartikel, der Vorrichtung zugeführt. Im Vordergrund, und damit im Gegensatz zu EP 0 504 459 A1, steht die Erzeugung einer ausreichend großen Zentrifugalkraft, die eine weitere Zerkleinerung der Partikel und damit eine Zerstäubung durch Aufprall der Partikel aufeinander und auf die Verneblerinnenwand bewirkt.

Auf demselben Prinzip hinsichtlich der Zerstäubung beruhend, arbeitet eine aus US 3.362.405 bekannte Vorrichtung, bei der ein pulverförmiger Stoff, der sich am Boden eines zylindrischen Behälters befindet, aufgewirbelt und durch Zusammenstöße der Partikel untereinander sowie mit der Behälterwand noch feiner zerrieben, d.h. zerstäubt wird. Die Vorrichtung dient nicht zur Pufferung, sondern zur Erzeugung eines trockenen Aerosols, das einfach an die Umgebung abgegeben wird. Die Erzeugung und die Abgabe erfolgen kontinuierlich und nicht in Abhängigkeit von der Atmung eines Benutzers. Bei der bekannten Vorrichtung wird die für die Zentrifugalkräfte erforderliche Wirbelströmung hervorgerufen, indem über ein tangential an der Mantelfläche des Zylinders angeordnetes Rohr Luft in den zylindrischen Behälter eingeblasen wird. Die aufgewirbelten Stoffpartikel werden durch Aufeinanderprallen sowie durch Wandimpaktion zerkleinert und gelangen zusammen mit der sie tragenden Luft aus dem zylindrischen Behälter durch ein Rohr hinaus, das mittig in der Stirnseite des Zylinders vorgesehen ist, in deren Nähe sich auch der Stutzen für die Zuluft befindet. Die Vorrichtung erinnert in Aufbau und Funktion an das Grundprinzip eines als "Zyklon" bekannten Abscheiders, da große Partikel des pulverförmigen Stoffes wieder abgeschieden werden.

Während sich die eine Gruppe der zuvor beschriebenen Vorrichtungen zur Behandlung eines nassen Aerosols eignet, scheidet die andere Gruppe der Vorrichtungen für diesen Zweck aus. Bei den zur Behandlung eines nassen Aerosols geeigneten Vorrichtungen sind aber Verbesserungen wünschenswert, insbesondere eine gezielte Beeinflussung der Aerosolpartikelgröße. Selbstverständlich sollen die verbesserten Vorrichtungen für Aerosole auf Pulverbasis geeignet sein; ferner soll weiterhin eine Ablagerung von Aerosolpartikeln und eine Verschlechterung des Ausräumungsgrades bei allen Aerosolarten vermieden werden.

Ausgehend von den zuerst beschriebenen Vorrichtungen liegt der Erfindung daher die Aufgabe zugrunde, eine Vorrichtung zur Trocknung und/oder Pufferung von Aerosolen zu schaffen, mit der die Eigenschaften des Aerosols, insbesondere bei nassen Aerosolen die Partikelgröße, gezielt beeinflußt werden kann, bei der die Ablagerung von Aerosolpartikeln vermieden wird und die grundsätzlich durch einen Atemzug vollständig ausgeräumt werden kann.

Gelöst wird diese Augfgabe durch eine Vorrichtung zur Trocknung und/oder Pufferung von Aerosolen mit den Merkmalen des Patentanspruchs 1. Vorteilhafte Ausgestaltungen ergeben sich aus den Unteransprüchen.

Die Erfindung nutzt in geschickter Weise die beiden bereits vom Zyklon-Abscheider bekannten Wirbel, die sich ineinander ausbilden. Dieser Vorgang wird bei der Schilderung der Ausführungsbeispiele der Erfindung noch genauer erläutert. Während aber bislang stets eine ausreichend hohe Rotationsgeschwindigkeit und damit eine hohe Zentrifugalkraft angestrebt wurde, zeichnet sich die erfindungsgemäße Vorrichtung im wesentlichen dadurch aus, daß aufgrund der geringeren Geschwindigkeit der äußere Wirbel eine Abschirmung bewirkt, die einerseits verhindert daß Aerosolpartikel die Gefäßwand erreichen und die anderseits den Behälter spült und damit eine sehr gute Ausräumung des Aerosols sicherstellt. Durch die Erfindung wird zur Trocknung und/oder Pufferung eines Aerosols, insbesondere eines nassen Aerosols der Einsatz einer im Aufbau an einen Zyklon-Abscheider erinnernden Vorrichtung vorgeschlagen, die durch die zentrale Zuführung des Aerosols von der einen Stirnseite und die atemzuggesteuerte Entnahme des Aerosols an der anderen Stirnseite des rotationsymmetrischen, vorzugsweise zylindrischen Grundkörpers unter Ausbildung der beiden ineinander liegenden Spiralwirbel eine gezielte Abtrocknung nasser Aerosole erlaubt, wobei eine Ablagerung an der Innenwand nahezu vermieden wird.

In einer bevorzugten Ausführungsform wird das Aerosol erzeugt durch eine Dispergierdüse, die in einem konisch zulaufenden Teil der zylindrischen Vorrichtung eingebracht ist. In dieser Düse wird eine Lösung und/oder Suspension mit trockener Luft vermischt und axial in die Vorrichtung eingesprüht. Die Betriebsparameter der Düse wie Dispergierluftfeuchte, Dispergierluftdurchsatz und Flüssigkeitsmassenstrom sind so auf die Abmessungen der Vorrichtung abgestimmt, daß minimale Impaktion am gegenüberliegenden Ende der Vorrichtung stattfindet (der Luftwechsel beim Füllen des Gefäßes entspricht nur etwa 80% des Volumens) und daß eine bestimmte mittlere Tröpfchengröße durch die Abtrocknung in der Dispergierluft erzielt werden kann. Das Gesamtvolumen der Vorrichtung ist so auf das Durchschnitts-Atemvolumen eines Patienten abgestimmt, daß die Vorrichtung in einem Atemzug problemlos ausgeräumt werden kann. Zwei bevorzugte Ausführungsformen haben daher jeweils ein Volumen von 570 ml (für Erwachsene) bzw. 350 ml (für zehnjährige Kinder).

In einer anderen bevorzugten Ausführungsform wird die unter Druck stehende Lösung oder Suspension in einem kurzen Stoß durch eine Einstoffdüse in die Vorrichtung eingespritzt, wobei die in der Vorrichtung enthaltene Luft nicht mit Lösungsmittel (Treibgas) gesättigt ist, sodaß eine Abtrocknung stattfinden kann. Aufgrund des kurzen Sprühstoßes können in diesem Fall das Volumen und die Länge der Vorrichtung weiter verkleinert bzw. verkürzt werden. Diese Ausführungsform hat dann ein Volumen von ungefähr 240 ml.

Inhaliert der Patient, so strömt durch den Sog, der über ein Tauchrohr in der der Düse gegenüberliegenden Seite der Vorrichtung auf das Aerosol im Inneren ausgeübt wird, Zuluft von oben in die Vorrichtung nach und wird dabei so geführt, daß Zyklonwirbel erzeugt werden, die an der Gefäßwand entlang in Richtung der Düse laufen. Diese Wirbel drängen das Aerosol zur Mitte des Gefäßes hin zusammen und versetzen das Aerosol in leichte Rotation. Die rotierende, innere Aerosolsäule wird dann durch das Tauchrohr abgesaugt und die Vorrichtung mit nachfließender Zuluft vollständig ausgespült. Dadurch wird eine sehr gute Ausräumung erzielt. Da die Bündelung und Führung des Aerosols allein durch Luftströmungen bewirkt wird, findet praktisch kein Aufprall auf feste Strömungsführungselemente statt, der einen Wirkstoffverlust durch Abscheidung verursachen würde.

Weitere Einzelheiten und Vorteile der Erfindung ergeben sich aus der Beschreibung der in den Zeichnungen dargestellten Ausführungsbeispiele der erfindungsgemäßen Vorrichtung. Die Zeichnungen zeigen:
Fig. 1 eine Perspektivansicht eines ersten, mit einer tangentialen Luftzuführung ausgestatteten Ausführungsbeispiels der erfindungsgemäßen Vorrichtung;
Fig. 2 die Strömungsverhältnisse in der erfindungsgemäßen Vorrichtung beim Vernebelungs- bzw. Abatmungsvorgang beispielhaft an dem ersten Ausführungsbeispiel;
Fig. 3 einen Längsschnitt durch die Vorrichtung des ersten Ausführungsbeispiels;
Fig. 4 eine Schnittansicht des stirnseitigen Endes des ersten Ausführungsbeispiels in der Höhe des tangentialen Zuluftrohrs;
Fig. 5 ein zweites Ausführungsbeispiel, bei dem die Zuluft über ein achsenparalleles Rohr zugeführt wird; und
Fig. 6 ein drittes Ausführungsbeispiel, bei dem die Zuluft radial der Vorrichtung zugeführt wird.

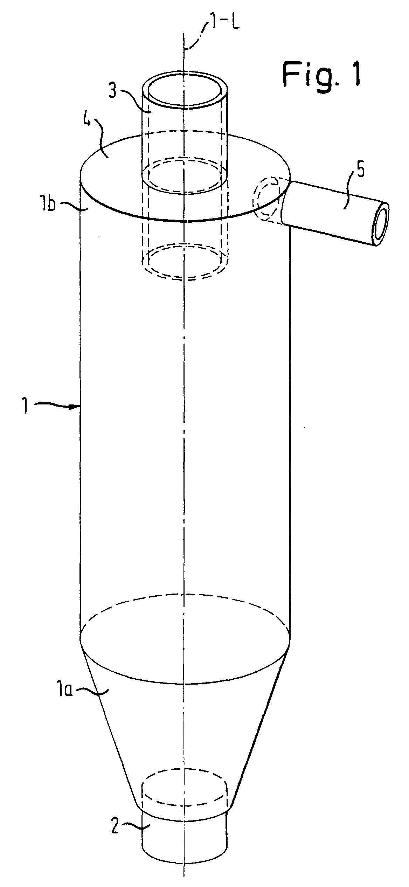

Die in Fig. 1 dargestellte Vorrichtung besteht aus einem zylindrischen Grundkörper 1, der sich an einem ersten stirnseitigen Ende in Form eines Kegelstumpfs 1a verjüngt. Die kleinere Öffnung des Kegelstumpfs 1a mit einem Durchmesser D₂ ist für den Anschluß einer Dispergierdüse 2 ausgebildet. Die Düse 2 wird abgedichtet eingesetzt, z.B. mit einem O-Ring (nicht abgebildet) und hat eine zur Symmetrieachse 1-L des zylindrischen Grundkörpers 1 koaxiale Einspritzrichtung. In der zweiten Stirnseite 1b des Grundkörpers 1 ist ein Rohrstück 3 durch ein Loch in der diese Stirnseite verschließenden Fläche 4 koaxial zur Rotationsachse 1-L des Grundkörpers 1 eingebracht, wobei das Rohrstück 3 um eine definierte Länge L₃ in den Grundkörper 1 hineinragt. Seitlich an das zweite stirnseitige Ende 1b des Grundkörpers 1 ist ein tangential einmündendes Rohrstück 5 angebracht, welches der Zuführung von Zuluft unter Ausbildung von Wirbeln um die Rotationsachse 1-L des Grundkörpers 1 dient.

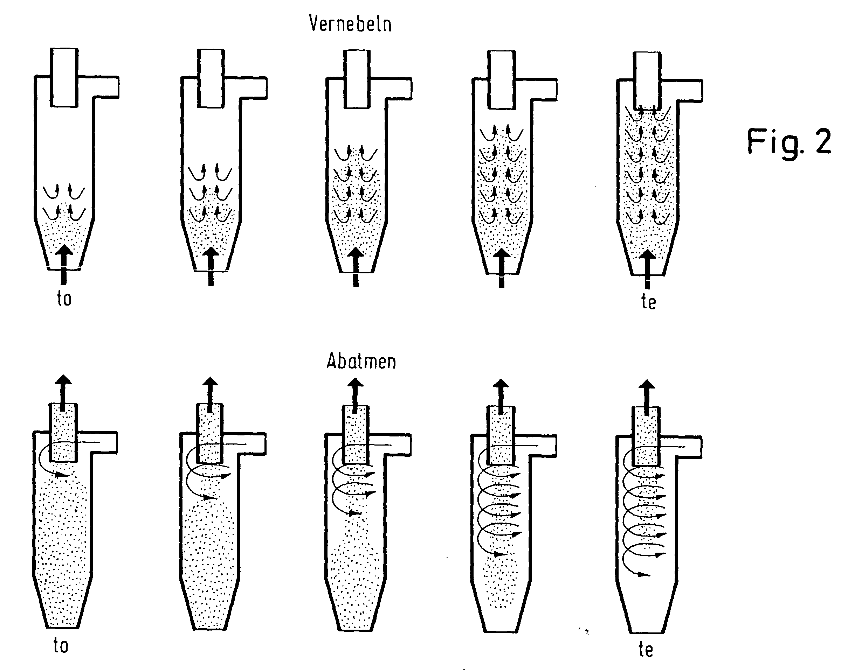

In der Dispergierdüse 2 wird eine Medikamentlösung mit trockener Luft vermischt und das derart erzeugte Ausgangsaerosol entlang der Symmetrieachse 1-L des Grundkörpers 1 zentral eingesprüht. Fig. 2 zeigt, wie der aufwärtsgerichtete Aerosolstrahl ein Hinabströmen von Luft entlang der Gefäßwand bewirkt. Die Summe dieser Strömungen führt zu Ring-Wirbeln, die ihre Rotationsachse senkrecht zur Symmetrieachse 1-L des Grundkörpers 1 haben. Diese Wirbel führen zu effizienter Verteilung des Aerosols und beruhigen es gleichzeitig, da den Aerosolpartikeln Bewegungsenergie entzogen wird. Dadurch kommt das Aerosol ins Gleichgewicht und die Partikel trocknen nach kurzer Zeit auf die gewünschte Größe ab. Die Düse 2 ist derart aufgebaut, daß das Aerosol nahe am Tauchrohr 3 bereits den oben beschriebenen Wirbeln strömungsmäßig folgt, um den Aufprall am oberen Ende 1b des Grundgefäßes 1 als Folge von Trägheitskräften auszuschließen, wodurch eine Ablagerung des Aerosols an dieser Stelle weitgehend vermieden wird.

Nachdem das Gefäß 1 gefüllt ist, d.h. der Einspritzvorgang abgeschlossen ist, kann das Aerosol über das Tauchrohr 3 abgeatmet werden. Zu diesem Zweck ist es günstig, ein Mundstück (nicht abgebildet) direkt auf das Tauchrohr aufzustecken und dies möglichst in gerader Verlängerung des Tauchrohrs 3 anzubringen, um Abscheidungsverluste zu vermeiden. Das Tauchrohr 3 vermittelt den durch das Abatmen entstehenden Sog an das Grundgefäß 1, wodurch Zuluft über das tangentiale Rohrstück 5 zuströmt. Die tangentiale Zuführung 5 gekoppelt mit dem zentralen Tauchrohr 3 führt zur Ausbildung von Zyklonwirbeln, die sich um die Rotationsachse des Grundkörpers 1 entlang der Gefäßwand nach unten schrauben, wie in Fig. 2 abgebildet.

Durch diese Wirbel wird das Aerosol zur Mitte des Gefäßes 1 hin auf einen Durchmesser, der ungefähr dem des Tauchrohrs 3 entspricht, zusammengedrängt, und durch Luftreibung leicht in Rotation versetzt. Dadurch, daß die durch Einatmen bewirkten Strömungsgeschwindigkeiten bei erfindungsgemäßer Gestaltung und Auslegung der Vorrichtung klein sind, wird ein übermäßiger Zentrifugaleffekt, wie einleitend beschrieben, verhindert. Gleichzeitig mit der Ausbildung der Zyklonwirbel wird die rotierende Aerosolsäule durch das Tauchrohr 3 abgesaugt und das Trocknergefäß 1 schließlich durch nachströmende Zuluft ausgespült. Danach kann wieder Aerosol eingesprüht werden und so ein neuer Inhalationszyklus begonnen werden.

Die Vorrichtung kann in allen räumlichen Lagen betrieben werden, sofern Befüllung und Abatmung zeitlich dicht aufeinander folgen (Sedimentation von Partikeln vernachlässigbar).

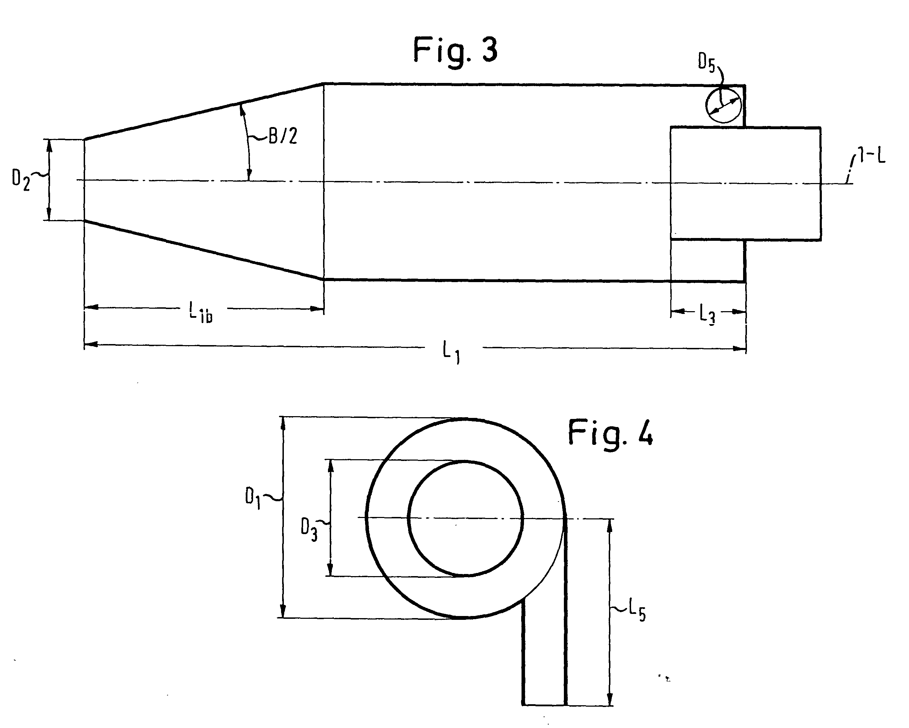

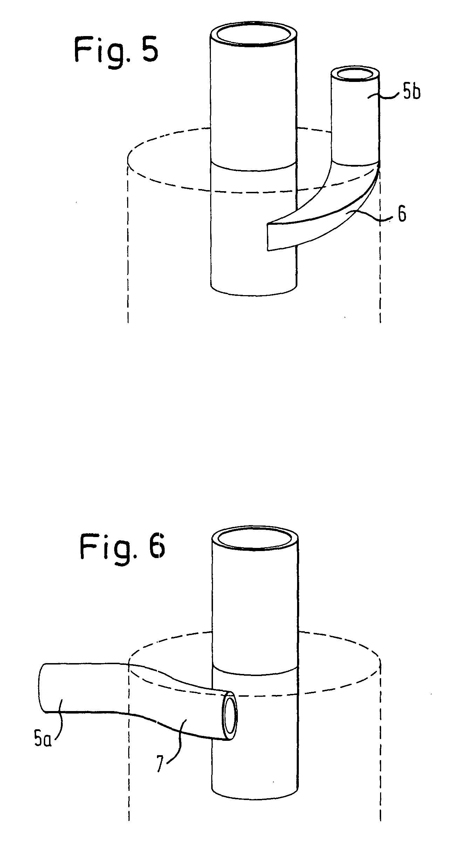

In den Fig. 3 und 4 sowie in nachstehender Tabelle sind zweckmäßige Abmessungen des in Fig. 1 dargestellten Ausführungsbeispiels veranschaulicht.

| Nr. | B [°] | D₁ [mm] | L₁ [mm] | D₃[mm] | L₃[mm] | D₅[mm] | L₅ [mm] | L_{1b} [mm] | D₂ [mm] | V [ml] |
|---|---|---|---|---|---|---|---|---|---|---|
| 1 | 28 | 54 | 180 | 30 | 20 | 10 | 50 | 65 | 22 | 350 |
| 2 | 28 | 64 | 210 | 40 | 25 | 10 | 50 | 85 | 22 | 570 |
| 3 | 28 | 64 | 120 | 40 | 25 | 10 | 50 | 85 | 22 | 240 |

Allgemein sollte der Durchmesser D₅ des Zuluftrohrs 5 vorzugsweise so gewählt werden, daß die Summe aus dem Tauchrohrdurchmesser D₃ und zweimal der lichten Weite D₅ des Zuluftrohres ungefähr gleich dem Durchmesser D₁ des Trocknergefäßes 1 ist. Mit anderen Worten, der Öffnungsdurchmesser D₅ des Zuluftrohres 5 sollte die neben dem Tauchrohr 3 zur Verfügung stehende Breite weitgehend ausnutzen. Dann nämlich erfolgt die zirkulare Strömungsführung um das Tauchrohr 3 ohne Aufweitung oder Einengung des aus dem Zuluftrohr 5 eintretenden Luftstroms, was eine optimale Strömung und Ausräumung begünstigt. Ferner ist es vorteilhaft, die in den Trockner hineinragende Länge L₃ des Tauchrohrs 3 doppelt so groß zu wählen wie den Durchmesser D₅ des Zuluftrohrs 5, da dadurch eine besonders gute Ausbildung der Zyklonwirbel gewährleistet ist. Länger sollte das Tauchrohr 3 nicht gewählt werden, da sich sonst beim Einspritzvorgang Aerosol durch verstärkte Impaktion am und im Tauchrohr 5 niederschlagen würde.

Das Gesamtvolumen der Vorrichtung ist vorzugsweise so auf das Atemvolumen einer Patientengruppe zu optimieren, daß sie möglichst mit einem Atemzug vollständig ausgeräumt werden kann. Aufgrund der empirisch festgestellten durchschnittlichen Atemzugvolumina von 750 ml für Erwachsene bzw. 450 ml für etwa 10jährige Kinder, hat eine bevorzugte Ausführungsform für Erwachsene ein Gesamtvolumen von 570 ml und eine weitere Ausführungsform für Kinder ein Volumen von 350 ml.

Bei der Spacervolumenauslegung wird ein 20 bis 30%iger Abschlag vom jeweiligen Atemzugvolumen berücksichtigt, damit die Mündhöhle und die oberen Atemwege, in denen das Aerosol keine Wirkung erzielen kann, mit Frischluft ausgewaschen werden können (z.B. 200 ml beim Erwachsenen).

In dem bislang beschriebenen Ausführungsbeispiel wird die Zuluft durch ein gerades, tangential einmündendes Rohrstück 5 zugeführt. Durch die direkte Führung ohne Umlenkeinrichtungen wird ein minimaler Strömungswiderstand erzielt, was bei Patienten mit einer Lungenschwäche sehr bedeutend ist, da dieser Widerstand beim Inhalieren überwunden werden muß. Es sind jedoch auch andere Ausführungsformen denkbar. So könnte, um Platz zu sparen, die Zuluftleitung auch zirkular an der Außenwand des zweiten stirnseitigen Endes 1b geführt werden (nicht abgebildet) oder auch ein achsen-parallel von oben einmündendes Rohrstück 5b gewählt werden, wie in Fig. 5 abgebildet. In letzterem Fall müßte aber zusätzlich eine den Strömungswiderstand erhöhende Umlenkeinrichtung zur Erzeugung einer tangentialen Strömung hinzugefügt werden. In Fig. 5 ist eine schaufelförmige Führungsfläche 6 abgebildet. Schließlich ist auch eine radiale Zulufteinführung 5a denkbar, wie in Fig. 6 abgebildet, mit einer angepaßten Umlenkeinrichtung 7 zur Erzeugung einer tangentialen Strömung.

Unabhängig von der speziellen Ausgestaltung der Zuluftführung ist es vorteilhaft, an dem der Gehäusemündung entgegengesetzten Ende des Zuluftrohrs ein Inspirationsventil (nicht abgebildet) anzubringen, um ein Herausströmen von Luft aus dem Grundgefäß 1 auf diesem Wege zu verhindern. Wichtig ist dann eine ausreichende Länge der Zuluftführung, damit so die vom Ventil verursachte turbulente Strömung bis zum Eintritt in das Gefäß 1 wieder vergleichmäßigt werden kann. Eine stark turbulente Einströmung oder ein asymmetrisches Strömungsprofil würde die Effizienz der Vorrichtung senken, da die Ausbildung von Zyklonwirbeln ungünstig beeinflußt würde und der Strömungswiderstand unnötig erhöht werden würde.

Der konische Zulauf des ersten stirnseitigen Endes 1a des Grundgefäßes 1, der in den Fig. 1 und 3 dargestellt ist, dient der Vermeidung von Toträumen, in denen sich Aerosol sammeln könnte ohne ausgeräumt zu werden. Die zylindrische Form des Grundkörpers 1 in den Ausführungsbeispielen ist die einfachste Form, die die Ausbildung von Zyklonwirbeln bei tangentialer Luftzuführung ermöglicht. Es sind aber auch andere rotationssymmetrische Grundkörper denkbar, so zum Beispiel ein gesamtkonischer Verlauf vom einen Ende 1a bis zum anderen Ende 1b (nicht abgebildet). In diesem Fall hätte das gesamte Gefäß (Grundkörper 1 und Ende 1a) die Form eines Kegelstumpfs.

Da durch die Reibung der strömenden Luft an der Gefäßwand der Vorrichtung eine elektrostatische Aufladung der Wand stattfinden kann, ist es vorteilhaft, das Gefäß aus einem antistatisch wirkenden Stoff zu fertigen. Die durch die Aufladung verursachten Anziehungskräfte würden eine verstärkte Ablagerung von Aerosolpartikeln an der Gefäßwand bewirken, was einen Verlust an Medikament bedeuten würde. Auch eine Beschichtung der Gefäßinnenwand mit einem antistatischen Belag ist denkbar.

Schließlich besteht noch ein vorteilhaftes Ausführungsbeispiel der in Fig. 1 dargestellten Vorrichtung darin, den zylindrischen Hauptkörper 1, den sich konisch verjüngenden Teil 1a sowie das das Tauchrohr 3 und das Zuluftrohr 5 enthaltende entgegengesetzte Ende 1b als separate Teile zu fertigen, die lösbar miteinander verbunden sind, z.B. durch einfaches Zusammenstecken. Dies vereinfacht die Herstellung der Vorrichtung, erleichtert aber auch die Reinigung und Desinfektion der Vorrichtung. Auch wäre dadurch eine Variation der Größe, beispielsweise durch Einsetzen eines verkürzten zylindrischen Zwischenstücks möglich, was einen Einsatz der Vorrichtung als kompakte Inhalationshilfe für mit einem Treibgas erzeugte Dosieraerosole ermöglichen würde.

## Patentansprüche

1. Vorrichtung zur Trocknung und/oder Pufferung von Aerosolen für Inhalationstherapiezwecke mit
- einem rotationssymmetrischen Grundkörper (1),
- der sich an einem ersten stirnseitigen Ende (1a) verjüngt und
- der an dem verjüngten stirnseitigen Ende (1a) für den Anschluß an eine Aerosolerzeugungseinrichtung (2) ausgebildet ist,
- einem Rohrstück (3) über das das Aerosol in dem Grundkörper (1) abgeatmet werden kann,
- das achsen-parallel zur Rotationsachse (1-L) des Grundkörpers (1)
- in einer der Grundkörper (1) an seinem zweiten stirnseitigen Ende (1b) verschließenden Fläche (4) angeordnet ist und
- das um eine definierte Länge (L₃) in den Grundkörper (1) hineinragt, und
- einem tangential in den Grundkörper (1) einmündenden Anschlußstutzen (5) für die Zuführung von Zuluft in den Grundkörper (1) unter Ausbildung von Wirbeln um die Rotationsachse (1-L) des Grundkörpers.

2. Vorrichtung nach Anspruch 1,
**dadurch gekennzeichnet,** daß
das Rohrstück (3) mittig in der das stirnseitige Ende (1b) verschließenden Fläche (4) angeordnet ist.

3. Vorrichtung nach Anspruch 2,
**dadurch gekennzeichnet**, daß
das tangential in den Grundkörper (1) einmündende Rohrstück (5) unmittelbar unterhalb der das stirnseitige Ende (1b) verschließenden Fläche (4) angeordnet ist.

4. Vorrichtung nach Anspruch 2 oder 3,
**dadurch gekennzeichnet**, daß
die Summe aus dem doppelten Durchmesser (D₅) des tangentialen Rohrstücks (5) und dem Durchmesser (D₃) des achsen-parallelen Rohrstücks (3) gleich dem Durchmesser (D₁) des Grundkörpers (1) ist.

5. Vorrichtung nach einem der Ansprüche 2 bis 4,
**dadurch gekennzeichnet**, daß
die Länge (L₃) mit der das achsen-parallele Rohrstück (3) in den Grundkörper hineinragt doppelt so groß ist wie der Durchmesser (D₅) des tangentialen Rohrstücks (5).

6. Vorrichtung nach Anspruch 1 oder 2,
**dadurch gekennzeichnet**, daß
der Anschlußstutzen (5) ein radial in den Grundkörper (1) einmündendes Rohrstück (5a) ist, an das sich eine Umlenkeinrichtung (7) anschließt.

7. Vorrichtung nach Anspruch 1 oder 2,
**dadurch gekennzeichnet**, daß
der Anschlußstutzen (5) ein achsen-parallel in den Grundkörper (1) einmündendes Rohrstück (5b) ist, an das sich eine Umlenkeinrichtung (6) anschließt.

8. Vorrichtung nach einem der Ansprüche 1 bis 7,
**dadurch gekennzeichnet**, daß
als Aerosolerzeugungseinrichtung (2) eine Zerstäuberdüse vorgesehen ist, deren Einspritzrichtung achsen-parallel ausgerichtet ist.

9. Vorrichtung nach einem der Ansprüche 1 bis 8,
**dadurch gekennzeichnet**, daß
das sich verjüngende stirnseitige Ende (1a) in Form eines Kegelstumpfs ausgebildet ist.

10. Vorrichtung nach einem der Ansprüche 1 bis 9,
**dadurch gekennzeichnet**, daß
der Grundkörper (1) ein Volumen von 570 ml, 350 ml oder 240 ml hat.

11. Vorrichtung nach einem der Ansprüche 1 bis 10,
**dadurch gekennzeichnet**, daß
der Anschlußstutzen (5) an seinem dem Grundkörper (1) abgewandten Ende mit einer Ventileinrichtung abgeschlossen ist.

12. Vorrichtung nach einem der Ansprüche 1 bis 11,
**dadurch gekennzeichnet**, daß
das Rohrstück (3) an seinem dem Grundkörper (1) abgewandten Ende mit einer Ventileinrichtung abschließt, an die sich ein Mundstück anschließt.

13. Vorrichtung nach einem der Ansprüche 1 bis 12,
**dadurch gekennzeichnet**, daß
Material und Oberfläche des Grundkörpers (1) so beschaffen sind, daß die Innenflächen des Grundkörpers (1) antistatisch sind.

14. Vorrichtung nach einem der Ansprüche 1 bis 13,
**dadurch gekennzeichnet**, daß
der Grundkörper (1), das erste stirnseitige Ende (1a) und das zweite stirnseitige Ende (1b) separate Teile sind, die lösbar miteinander verbunden sind.

## Claims

1. Device for drying and/or buffering aerosols for purposes of inhalation therapy having
- a rotationally symmetrical base structure (1)
- which tapers at a first face end (1a) and
- is constructed at the end of the tapering face end (1a) for the attachment of an aerosol generator device (2),
- a length of tube (3) through which the aerosol in the base structure (1) can be breathed down
- which axially parallel to the axis of rotation (1-L) of the base structure (1)
- is arranged in a surface (4) closing off the base structure (1) at its second face end (1b) and
- which projects by a defined length (L₃) into the base structure (1), and
- a connecting piece (5) opening out tangentially into the base structure(1) for the supply of incoming air into the base structure (1) with the formation of vortices around the axis of rotation (1-L) of the base structure.

2. Device according to Claim 1, **characterised in that** the length of tube (3) is arranged centrally in the surface (4) closing off the face end (1b).

3. Device according to Claim 2, **characterised in that** the length of tube (5) opening out tangentially into the base structure (1) is arranged directly below the surface (4) closing off the face end (1b).

4. Device according to Claim 2 or 3, **characterised in that** the sum of twice the diameter (D₅) of the tangential length of tube (5) and the diameter (D₃) of the axially parallel length of tube (3) equals the diameter (D₁) of the base structure (1).

5. Device according to one of Claims 2 to 4, **characterised in that** the length (L₃) by which the axially parallel length of tube (3) projects into the base structure is twice as great as the diameter (D₅) of the tangential length of tube (5).

6. Device according to Claim 1 or 2, **characterised in that** the connecting piece (5) is a length of tube (5a) opening out radially into the base structure (1) to which a diverting device (7) is connected.

7. Device according to Claim 1 or 2, **characterised in that** the connecting piece (5) is a length of tube (5b) opening out in axially parallel manner into the base structure (1) to which a diverting device (6) is connected.

8. Device according to one of Claims 1 to 7, **characterised in that** for the aerosol generation device (2) an atomising nozzle is provided whose injection direction is aligned in axially parallel manner.

9. Device according to one of Claims 1 to 8, **characterised in that** the tapering face end (1a) is constructed in the shape of a truncated cone.

10. Device according to one of Claims 1 to 9, **characterised in that** the base structure (1) has a volume of 570 ml, 350 ml or 240 ml.

11. Device according to one of Claims 1 to 10, **characterised in that** the connecting piece (5) is closed off at its end facing away from the base structure (1) by a valve device.

12. Device according to one of Claims 1 to 11, **characterised in that** the length of tube (3) terminates at its end facing away from the base structure (1) in a valve device to which a mouthpiece is connected.

13. Device according to one of Claims 1 to 12, **characterised in that** the material and surface of the base structure (1) are of such a nature that the internal surfaces of the base structure (1) are antistatic.

14. Device according to one of Claims 1 to 13, **characterised in that** the base structure (1), the first face end (1a) and the second face end (1b) are separate parts which are connected to one another in detachable manner.

## Revendications

1. Dispositif pour le séchage et/ou le tamponnage d'aérosols destinés à une thérapie par inhalation, comportant :
- un corps de base (1) à symétrie de rotation
- qui se rétrécit à une première extrémité frontale (1a), et
- qui est configuré à son extrémité frontale rétrécie (1a) pour le raccordement à un dispositif (2) de production d'un aérosol,
- une pièce tubulaire (3) par laquelle l'aérosol présent dans le corps de base (1) peut être inhalé,
- dont l'axe est parallèle à l'axe de rotation (1-L) du corps de base (1),
- qui est disposée dans une surface (4) qui ferme le corps de base (1) à sa deuxième extrémité frontale (1b), et
- qui pénètre dans le corps de base (1) sur une longueur définie (L3), et
- un raccord (5) qui débouche tangentiellement dans le corps de base (1), pour admettre de l'air dans le corps de base (1) avec formation de tourbillons autour de l'axe de rotation (1-L) du corps de base.

2. Dispositif selon la revendication 1, **caractérisé en ce que** la pièce tubulaire (3) est disposée au centre de la surface (4) qui ferme l'extrémité frontale (1b).

3. Dispositif selon la revendication 2, **caractérisé en ce que** la pièce tubulaire (5) qui débouche tangentiellement dans le corps de base (1) est disposée immédiatement en dessous de la surface (4) qui ferme l'extrémité frontale (1b).

4. Dispositif selon la revendication 2 ou 3, **caractérisé en ce que** la somme du double du diamètre (D5) de la pièce tubulaire tangentielle (5) et du diamètre (D3) de la pièce tubulaire (3) parallèle à l'axe est égale au diamètre (D1) du corps de base (1).

5. Dispositif selon l'une des revendications 2 à 4, **caractérisé en ce que** la longueur (L3) de laquelle la pièce tubulaire (3) parallèle à l'axe pénètre dans le corps de base vaut le double du diamètre (D5) de la pièce tangentielle (5).

6. Dispositif selon la revendication 1 ou 2, **caractérisé en ce que** le raccord (5) est une pièce tubulaire (5a) qui débouche radialement dans le corps de base (1) et à laquelle se raccorde un dispositif de changement de direction (7).

7. Dispositif selon la revendication 1 ou 2, **caractérisé en ce que** le raccord (5) est une pièce tubulaire (5b) qui débouche parallèlement à l'axe dans le corps de base (1) et à laquelle se raccorde un dispositif de changement de direction (6).

8. Dispositif selon l'une des revendications 1 à 7, **caractérisé en ce qu**'une buse de pulvérisation dont la direction de projection est orientée parallèlement à l'axe, est prévue comme dispositif (2) de production d'un aérosol.

9. Dispositif selon l'une des revendications 1 à 8, **caractérisé en ce que** l'extrémité frontale rétrécie (1a) est configurée en forme de tronc de cône.

10. Dispositif selon l'une des revendications 1 à 9, **caractérisée en ce que** le corps de base (1) a un volume de 570 ml, 350 ml ou 240 ml.

11. Dispositif selon l'une des revendications 1 à 10, **caractérisé en ce que**, à son extrémité non tournée vers le corps de base (1), le raccord (5) est fermé par un dispositif à soupape.

12. Dispositif selon l'une des revendications 1 à 11, **caractérisé en ce que**, à son extrémité non tournée vers le corps de base (1), la pièce tubulaire (3) est fermée par un dispositif à soupape auquel se raccorde un embout.

13. Dispositif selon l'une des revendications 1 à 12, **caractérisé en ce que** le matériau et la surface du corps de base (1) sont d'une nature telle que les surfaces intérieures du corps de base (1) sont antistatiques.

14. Dispositif selon l'une des revendications 1 à 13, **caractérisé en ce que** le corps de base (1), la première extrémité frontale (1a) et la deuxième extrémité frontale (1b) sont des pièces séparées qui sont reliées entre elles de manière libérable.
